# EUROPEAN PATENT SPECIFICATION

(11) **EP 4 134 440 B1**
(45) Date of publication and mention of the grant of the patent: **12.02.2025**
(21) Application number: 21784192.3
(22) Date of filing: 06.04.2021
(51) Int. Cl.: A61K 8/9728, A61K 8/9789, A61K 35/744, A61K 35/747, A61K 36/185, A61P 17/00, A61P 17/18, A61P 43/00, A61Q 17/04, A61Q 19/02, C12N 1/20, C12P 1/04, C12R 1/01, C12R 1/225, C12R 1/46

(54) **MALVA SYLVESTRIS FERMENTATION PRODUCT AND USE THEREOF**
MALVA SYLVESTRIS FERMENTATIONSPRODUKT UND VERWENDUNG DAVON
PRODUIT DE FERMENTATION DE MALVA SYLVESTRIS ET SON UTILISATION

(30) Priority: 09.04.2020 JP 2020070364
(43) Date of publication of application: 15.02.2023
(73) Proprietor: Kabushiki Kaisha Yakult Honsha, Tokyo, 105-8660 (JP)
(72) Inventor: KURASAWA, Tomoko, Tokyo 105-8660 (JP); SHIBATA, Shinya, Tokyo 105-8660 (JP); IZAWA, Naoki, Tokyo 105-8660 (JP); ITO, Masahiko, Tokyo 105-8660 (JP)
(74) Representative: Mewburn Ellis LLP
(86) International application number: PCT/JP2021/014617
(87) International publication number: WO 2021/206086

(56) References cited:
- JP-A- 2002 193 735
- JP-A- 2012 219 093
- JP-A- 2016 020 362
- JP-A- 2017 001 935
- US-A1- 2004 096 418
- DATABASE WPI Week 200860, Derwent World Patents Index; AN 2008-K18490, XP002811228
- DATABASE GNPD [online] MINTEL; 1 February 2017 (2017-02-01), ANONYMOUS: "One Minute Wrinkle Lift", XP093142274, retrieved from https://www.gnpd.com/sinatra/recordpage/4663409/ Database accession no. 4663409

## Description

### TECHNICAL FIELD

The present invention relates to a plant fermentation product useful as a topical skin agent, etc., more specifically to a *Malva sylvestris* fermentation product and a topical skin agent containing the same.

### BACKGROUND ART

*Malva sylvestris* (sometimes also called common mallow) is a perennial wild species native to Europe but is widely cultivated as an herb and for herbal medicines. Plant extracts and fermentation products have been used in the past in cosmetics, etc., but use as a topical skin agent has been reported for *Malva sylvestris.* Specifically, for example, Patent Document 1 describes the suppression of overexpression of bFGF mRNA, the expression of which is increased by UV irradiation, by *Malva sylvestris* extract. The possibility of preventing, treating, or ameliorating blemishing, freckling, skin pigmentation, etc., thereby is described. Also, for example, Patent Document 2 describes the suppression of the kinesin expression level in human epithelium-derived pigment cells by *Malva sylvestris* extract (common mallow extract). A beneficial effect on blemishes, freckles, and pigmentation is also described.

### [Related Art Documents]

### [Patent Documents]

[Patent Document 1] Japanese Laid-open Patent Application No. 2011-1328
[Patent Document 2] Japanese Laid-open Patent Application No. 2012-219091

### SUMMARY OF THE INVENTION

### [Problems the Invention is Intended to Solve]

However, methods using conventional extracts have been unable to adequately elicit activity by *Malva sylvestris.*

Therefore, an object of the present invention is to provide a *Malva sylvestris*-derived material modified to increase activity. Another purpose is to provide a novel topical skin agent thereby.

### [Means for Solving the Problems]

The inventors perfected the invention upon conducting thoroughgoing studies to attain the aforestated object.

The present invention according to a first aspect provides a fermentation product, using *Malva sylvestris* or a processed product thereof as a starting material, obtained using one or more microorganisms selected from the group consisting of microorganisms belonging to the genus Lactobacillus, microorganisms belonging to the genus Lactococcus, and microorganisms belonging to the genus Pediococcus.

In the fermentation product according to the present invention, the microorganisms are preferably one or more microorganisms selected from the group consisting of *Lactobacillus plantarum, Lactobacillus pentosus, Lactobacillus zeae, Lactobacillus mali, Lactobacillus fabifermentans, Lactobacillus hordei, Lactococcus lactis, Pediococcus acidilactici,* and *Pediococcus pentosaceus.*

The present invention according to a second aspect provides a topical skin agent containing the abovementioned fermentation product.

In the topical skin agent according to the present invention, the topical skin agent is preferably used to cause a whitening effect.

In the topical skin agent according to the present invention, the fermentation product preferably has a melanin production-suppressing action.

In the topical skin agent according to the present invention, the fermentation product preferably has a tyrosinase-inhibiting action.

### [Effect of the Invention]

The present invention has exceptional activity that causes a whitening effect such as melanin production-suppressing activity, tyrosinase-inhibiting activity, etc., due to being a fermentation product having *Malva sylvestris* or a processed product thereof as the starting material, treated by specific microorganisms. Therefore, a novel topical skin agent can be provided thereby.

### BRIEF DESCRIPTION OF THE DRAWINGS

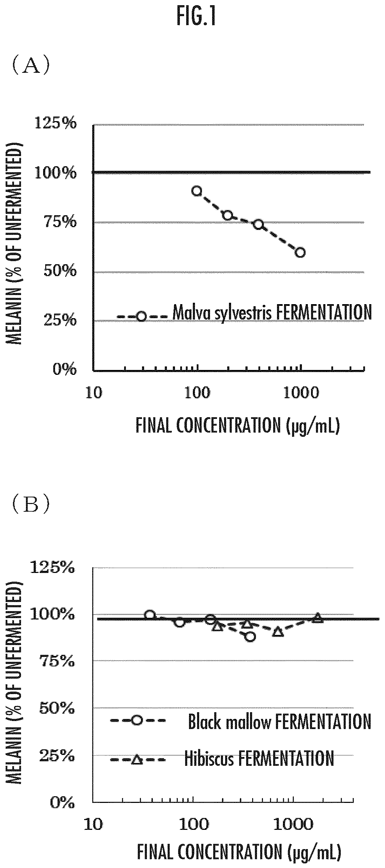

FIG. 1 is a graph showing the results of studying melanin production-suppressing activity in Test Case 4. (A) is a graph showing the results of studying the changes in the intracellular melanin production level at each final concentration of *Malva sylvestris* fermentation product added to melanin-producing cells (the results are shown as a percentage to the intracellular melanin level when unfermented *Malva sylvestris* extract was added in the same final concentrations), and (B) is a graph showing the results of the same test of hibiscus or black mallow.

### BEST MODE FOR CARRYING OUT THE INVENTION

In the present specification, *"Malva sylvestris"* is synonymous with the plant commonly understood by those skilled in the art and specifically includes in the meaning *Malva sylvestris* of the genus Malva, family Malvaceae (scientific name: *Malva sylvestris,* also called blue mallow) and a variety thereof *Malva mauritiana* (scientific name: *Malva mauritiana,* also called common mallow). *Malva sylvestris* is a plant of European origin, is also widely cultivated as an herb and for medicinal use, and is easily available.

In the present invention, microorganisms are allowed to act on *Malva sylvestris* to make a fermentation product by the microorganisms. Examples of the plant parts of *Malva sylvestris* on which the microorganisms act include the flowers, leaves, stems, aerial portions, roots, whole plant, a mixture of these parts, etc. However, the flowers, leaves, or a mixture of these parts, etc., are preferred, and the flowers are more preferred. The form of the plant on which the microorganisms act is a crushed product of the bulk plant or a dried product thereof, a juice, an extract, or a mixture of these, etc., but no particular limitations are placed thereon. A juice, extract, or a mixture of these, etc., is preferred, and an extract is more desirable.

Examples of the microorganisms that act upon *Malva sylvestris* include microorganisms belonging to the genus Lactobacillus, microorganisms belonging to the genus Lactococcus, microorganisms belonging to the genus Leuconostoc, microorganisms belonging to the genus Pediococcus, etc.; more specifically, *Lactobacillus plantarum, Lactobacillus pentosus, Lactobacillus zeae, Lactobacillus mali, Lactobacillus fabifermentans, Lactobacillus hordei, Lactococcus lactis, Pediococcus acidilactici, Pediococcus pentosaceus,* etc. With these microorganisms, fermentation of *Malva sylvestris* is promoted well, and the activity that causes a whitening effect is excellent. However, this does not mean that microorganisms that can be used in the present invention are limited to these species. One type of microorganism may be used alone in treatment with the above starting material, or two or more types may be used in combination in treatment with the above starting material. Specifically, treatment by two or more different types of microorganisms may be carried out sequentially, or treatment may be carried out using two or more different types of microorganisms in combination simultaneously. Alternatively, these treatments may be combined.

The conditions under which the microorganisms act upon *Malva sylvestris* are not particularly limited as long as the conditions cause some type of change in the components of *Malva sylvestris* by the microorganisms. However, growth conditions under which the microorganisms that act upon *Malva sylvestris* proliferate, for example, 2-10,000 times the starting cell count, typically 5-1000 times, and more typically 10-1000 times, are preferred. When growth is poor, fermentation does not progress well.

When allowing the microorganisms to act on *Malva sylvestris,* for example, glucose, fructose, sucrose, oligosaccharides, and other such sugars; alanine, arginine, tryptophan, cysteine, and other such amino acids; casein degradation product, protein degradation product, and other such peptides; yeast extract, meat extract, soybean extract, and other such extracts; polyoxyethylene sorbitan oleate and other such surfactants having a fatty acid in a side chain; or, for example, Lactobacillus MRS Broth (Difco), etc., which is a standard medium composition for lactobacilli, etc., may be used as auxiliary materials for growth of the microorganisms.

However, if other components remain in addition to *Malva* sylvestris-derived components, the quality of the fermentation product obtained is sometimes affected in terms of the antiseptic property, feel on use, etc. Therefore, it is not desirable to combine more components other than *Malva sylvestris*-derived components than necessary during fermentation. Consequently, when using the auxiliary materials, the proportion of other components relative to 100 mass parts of *Malva* sylvestris-derived components is preferably set at from 0.001 mass part to 5.0 mass parts, more preferably from 0.01 mass part to 0.5 mass part. On the other hand, *Malva sylvestris* may be subjected to treatment by the microorganisms without adding a starting material not derived from *Malva sylvestris.*

Discretionary, non-limiting embodiments for obtaining a fermentation product according to the present invention are explained more specifically below.

As the starting material upon which the microorganisms act, for example, an extract of *Malva sylvestris* can be obtained and used as the starting material. In this case, for example, a 10- to 200-fold amount of water or hot water (for example, reverse osmosis-treated water, ion-exchanged water, tap water, well water, distilled water, ultrapure water, etc., may be used) may be added to a dried powder of the plant and heated to obtain a hot-water extract. The hot-water extract may be used unmodified as the starting material in the form of an extract suspension, although optionally the water may be evaporated off to effect concentration for use as the starting material, or the solids may be removed by a solid-liquid separation means such as filter filtration, centrifugation, etc., to obtain a supernatant to use as the starting material. Such a starting material may be inoculated with the microorganisms in a suitable starting concentration so that the microorganisms act upon the starting material.

Treatment by the microorganisms can be carried out by methods according with normal aeration and static culture. In this case, the starting cell count concentration is preferably from 1 × 10⁴ CFU/mL to 5 × 10⁷ CFU/mL, more preferably 1 × 10⁵ CFU/mL to 1 × 10⁷ CFU/mL. The temperature conditions are 20°C-40°C, more preferably 25°C-37°C. The treatment time is from 12 hours to 10 days, more preferably 1-3 days. By static culture under such conditions, for example, the microorganisms are caused to proliferate to 2-10,000 times the starting cell count, typically 5-1000 times, more typically 10-1000 times, and a fermentation product can be obtained. After treatment by the microorganisms, the fermentation product according to the present invention may be used directly, including the microorganisms used, but it is preferable in terms of the antiseptic property, feel on use, etc., as mentioned above to remove the cells of the microorganisms used in fermentation by a solid-liquid separation means such as filter filtration, centrifugation, etc., and use the supernatant obtained as the fermentation product. It is also possible after treatment by the microorganisms to add various solvents to the treated product to prepare an extract or a diluted product which is used as the fermentation product of the present invention. Examples of solvents used here include, but are not limited to, solvents commonly used in cosmetics such as water; ethanol, propanol, and other such lower alcohols; cetyl alcohol, stearyl alcohol, and other such higher alcohols; 1,3-butylene glycol, 1,3-propanediol, glycerin, and other such polyhydric alcohols; etc. The solvents can be used individually or in mixtures of two or more.

The fermentation product according to the present invention may be used without modification as a topical skin agent or may be used by being blended in a topical skin agent production process. Specifically, the fermentation product can be used suitably, for example, as a cosmetic in the form of an emulsion, cream, cleanser, massage, sunscreen, foundation, cream foundation, etc., or as a raw material thereof. Furthermore, the term cosmetic as used here includes pharmaceuticals, quasi-drugs, and cosmetics defined by the Act on Securing Quality, Efficacy, and Safety of Products Including Pharmaceuticals and Medical Devices.

Also, the form of a topical skin agent may be a pack, mask, gel, etc., in which a component that acts on the skin is carried by a suitable base material, in other words, a form that can be used suitably as a component that acts on the skin in such a topical skin agent.

Also, in discretionary, non-limiting embodiments of the present invention, the topical skin agent may be used to cause a whitening effect or may be a product that exhibits functionalities such as having a melanin production-suppressing action, having a tyrosinase-inhibiting action, etc.

### EXAMPLES

The present invention is explained concretely below through examples, but these examples do not limit the scope of the present invention.

### [1. Preparation of Malva sylvestris extract]

### 1-1. Plant

Commercial dried flowers of *Malva sylvestris* were used in testing.

### 1-2. Extraction

*Malva sylvestris* extract for preculture was prepared as follows. Specifically, water (reverse osmosis-treated water, referred to hereinafter as "RO water") was added so that the plant powder:water ratio was 1:20 (mass). Glucose was added to yield a final concentration of 0.2 w/v% and yeast extract (Difco) was added to yield a final concentration of 0.1 w/v%. A suspension was prepared via thorough stirring. The suspension was aliquoted in 3 mL portions into test tubes which were then covered with an aluminum cap and autoclaved for 15 minutes at 121°C to obtain a hot-water extract.

Two types of *Malva sylvestris* extract for main culture were prepared: (1) no other ingredients added, and (2) 0.2 w/v% glucose and 0.1 w/v% yeast extract added. Specifically, RO water was added so that the plant powder:water ratio was 1:20 (mass). For type (1), no other ingredients were added, while for type (2), glucose was added to yield a final concentration of 0.2 w/v% and yeast extract (Difco) was added to yield a final concentration of 0.1 w/v%. Suspensions were prepared via thorough stirring. The suspensions were aliquoted in 10 mL portions into test tubes which were sealed with a silicone plug and autoclaved for 100 minutes at 98°C to obtain a hot-water extract.

### [2. Lactic acid bacteria]

Table 1 shows the 13 types of lactic acid bacteria used. -80°C DMSO preserved strains were inoculated onto Lactobacilli MRS Broth (Difco). After culturing for 20 hours at the optimal culture temperature, cells passaged for another generation under the same conditions were formed into a bacterial solution and provided for preculture of *Malva sylvestris* extract. Furthermore, a type of strain representing the genus and species of each of the lactic acid bacteria was obtained and used for 11 of the lactic acid bacteria used (other than No. 5 and No. 11). The 13 strains used can be obtained from the depositories listed in Table 1.

**[Table 1]**

| Lactic acid bacterium No. | T: Type strain*¹ | Genus and species | ATCC*² No. | JCM*³ No. | NBRC*⁴ No. | DSM*⁵ No. | FERM*⁶ No. | Optimal culture temperature (°C) |
|---|---|---|---|---|---|---|---|---|
| 1 | T | *Lactobacillus plantarum* subsp. *plantarum* | ATCC 14917 | JCM 1149 | NBRC 15891 | DSM 20174 | | 30 |
| 2 | T | *Lactobacillus pentosus* | ATCC 8041 | JCM 1558 | NBRC 106467 | DSM 20314 | | 30 |
| 3 | T | *Lactobacillus zeae* | ATCC 15820 | JCM 11302 | | DSM 20178 | | 37 |
| 4 | T | *Lactobacillus mali* | ATCC 27053 | JCM 1116 | NBRC 102159 | DSM 20444 | | 30 |
| 5 | | *Lactobacillus casei* | | | | | FERM-BP 1366 | 37 |
| 6 | T | *Lactobacillus fabifermentans* | | | | DSM 21115 | | 30 |
| 7 | T | *Lactobacillus hordei* | | JCM 16179 | | DSM 19519 | | 30 |
| 8 | T | *Lactococcus lactis* subsp. *lactis* | ATCC 19435 | JCM 5805 | NBRC 100933 | DSM 20481 | | 30 |
| 9 | T | *Leuconostoc pseudomesenteroides* | ATCC 12291 | JCM 9696 | | DSM 20193 | | 30 |
| 10 | | Leuconostoc *mesenteroides* subsp. *mesenteroides* | ATCC 14430 | | | | | 26 |
| 11 | T | *Pediococcus acidilactici* | ATCC 33314 | JCM 5885 | | DSM 20333 | | 37 |
| 12 | T | *Pediococcus pentosaceus* | ATCC 33316 | JCM 5890 | NBRC 107768 | DSM 20336 | | 37 |
| 13 | T | *Streptococcus thermophilus* | ATCC 19258 | JCM 17834 | NBRC 111149 | DSM 20617 | | 37 |

| | | | | | | | | |
|---|---|---|---|---|---|---|---|---|
| Type strain*¹: type strain ATCC*²: American Type Culture Collection JCM*³: Riken BioResource Research Center, Microbe Division NBRC*⁴: NITE Biological Resource Center DSM*⁵: German Collection of Microorganisms and Cell Cultures FERM*⁶: NITE International Patent Organism Depository | | | | | | | | |

### [Test Example 1]

In preculture, 3 mL of *Malva sylvestris* extract for preculture was inoculated with 0.5 v/v% bacterial solution (starting cell count concentration: approximately 0.5 × 10⁶ to 2 × 10⁷ CFU/mL) and static cultured for 48 hours at the optimal temperature under an aerobic condition.

In main culture, 10 mL of *Malva sylvestris* extract for main culture was inoculated with 1 v/v% precultured culture (starting cell count concentration: approximately 0.5 × 10⁵ to 5 × 10⁶ CFU/mL) and static cultured for 72 hours at the optimal temperature under an aerobic condition.

After main culture, the viable cell count was confirmed. Specifically, 100 µL of an undiluted solution of the main cultured culture or a solution obtained by suitably diluting the main cultured culture by 0.1 w/v% yeast extract was seeded on Lactobacilli MRS agar plate medium using a spiral plater EDDY JET2 (IUL Instruments). After culturing for three days at the optimal temperature, the colonies formed were counted using a colony counter ProtoCOL3 (SYNBIOSIS), and the value of CFU (colony-forming units)/mL was calculated. The starting cell count before main culture was confirmed in the same way.

**[Table 2]**

| Lactic acid bacterium No. | Genus and species | Medium: *Malva sylvestris* extract | | | |
|---|---|---|---|---|---|
| | | Unadded | | GY added* | |
| | | Starting cell count | Viable cell count attained | Starting cell count | Viable cell count attained |
| | | CFU (× 10⁶/mL) | | CFU (× 10⁶/mL) | |
| 1 | *Lactobacillus plantarum* subsp. *plantarum* | 2.1 | 40 | 2.1 | 60 |
| 2 | *Lactobacillus pentosus* | 3.2 | 240 | 3.2 | 120 |
| 3 | *Lactobacillus zeae* | 1.8 | 140 | 1.8 | 63 |
| 4 | *Lactobacillus mali* | 3.7 | 64 | 3.7 | 82 |
| 5 | *Lactobacillus casei* | 1.6 | 25 | 1.6 | 53 |
| 6 | *Lactobacillus fabifermentans* | 0.46 | 11 | 0.46 | 22 |
| 7 | *Lactobacillus hordei* | 2.0 | 57 | 2.0 | 140 |
| 8 | *Lactococcus lactis* subsp. *lactis* | 0.053 | 5.4 | 0.053 | 5 |
| 9 | *Leuconostoc pseudomesenteroides* | 0.42 | 45 | 0.42 | 8 |
| 10 | *Leuconostoc mesenteroides* subsp. *mesenteroides* | 4.4 | 66 | 4.4 | 93 |
| 11 | *Pediococcus acidilactici* | 0.23 | 41 | 0.23 | 20 |
| 12 | *Pediococcus pentosaceus* | 0.5 | 24 | 0.5 | 34 |
| 13 | *Streptococcus thermophilus* | ND** | -- | ND** | -- |

| | | | | | |
|---|---|---|---|---|---|
| GY added*: 0.2 w/v% glucose + 0.1 w/v% yeast extract ND**: not detected after preculture | | | | | |

As a result, lactic acid bacterium No. 13 (*Streptococcus thermophilus*) was a species or strain unsuited to preparation of a fermentation product using *Malva sylvestris* as the starting material because no viable bacteria were detected after preculture.

With the other 12 strains of lactic acid bacteria, the viable cell count increased at least ten-fold over the starting cell count due to culture in *Malva sylvestris* extract. The effects when glucose and yeast extract were added to *Malva sylvestris* extract varied depending on the type of lactic acid bacterium, with the viable cell count tending to increase (e.g., lactic acid bacterium No. 7 (*Lactobacillus hordei)),* remaining basically the same (e.g., lactic acid bacterium No. 8 (*Lactococcus lactis* subsp. *lactis*)), and tending to suppression (e.g., lactic acid bacterium No. 3 (*Lactobacillus zeae*)). No uniform trend was seen. The magnitude of the effect on growth of the bacteria was not that remarkable in any case.

The above clarified that the 12 strains of lactic acid bacteria, that is, lactic acid bacterium No. 1 *(Lactobacillus plantarum* subsp. *plantarum),* lactic acid bacterium No. 2 *(Lactobacillus pentosus),* lactic acid bacterium No. 3 *(Lactobacillus zeae),* lactic acid bacterium No. 4 *(Lactobacillus mali),* lactic acid bacterium No. 5 *(Lactobacillus casei),* lactic acid bacterium No. 6 *(Lactobacillus fabifermentans),* lactic acid bacterium No. 7 *(Lactobacillus hordei),* lactic acid bacterium No. 8 *(Lactococcus lactis* subsp. *lactis),* lactic acid bacterium No. *9 (Leuconostoc pseudomesenteroides*), lactic acid bacterium No. *10 (Leuconostoc mesenteroides* subsp. *mesenteroides*), lactic acid bacterium No. 11 (*Pediococcus acidilactici),* and lactic acid bacterium No. 12 (*Pediococcus pentosaceus),* are suited to preparation of a fermentation product using *Malva sylvestris* as the starting material.

### [Test Example 2]

Lactic acid bacteria Nos. 1, 2, 4, 6, 9, and 10 were selected from among the 12 types of lactic acid bacteria (Nos. 1-12) that exhibited a viable cell count after culture of 1 × 10⁶ CFU/mL or more in Test Example 1, and melanin production-suppressing activity was studied using cultures obtained by each lactic acid bacterium.

Specifically, after main culture by unadded *Malva sylvestris* extract had been completed, the culture was centrifuged for 10 minutes at 3000 rpm (1600 × g), and the supernatant was aseptically filtered by a 0.22 µm filter to obtain a fermentation supernatant. The fermentation supernatant was stored at 4°C shielded from light, then stored frozen at -20°C until measurement. Table 3 shows the results of pH measurement of the fermentation supernatant used or unfermented *Malva sylvestris* extract and the evaporation residue concentration (mg/mL) when measured by allowing to cool in a desiccator after heating for three hours at 105°C.

**[Table 3]**

| Sample No. | Type of sample | pH | Evaporation residue concentration* (mg/mL) |
|---|---|---|---|
| 1 | Fermentation supernatant by lactic acid bacterium No. 1 | 3. 9 | 20.0 |
| 2 | Fermentation supernatant by lactic acid bacterium No. 2 | 4.1 | 19.7 |
| 4 | Fermentation supernatant by lactic acid bacterium No. 4 | 4.1 | 19.6 |
| 6 | Fermentation supernatant by lactic acid bacterium No. 6 | 3. 9 | 21.2 |
| 9 | Fermentation supernatant by lactic acid bacterium No. 9 | 4.5 | 20.6 |
| 10 | Fermentation supernatant by lactic acid bacterium No. 10 | 4.3 | 21.0 |
| 14 | *Malva sylvestris* extract (unfermented) | 5.1 | 21.8 |

| | | | |
|---|---|---|---|
| Evaporation residue concentration*: allowed to cool in desiccator after heating for three hours at 105°C | | | |

Melanin production-suppressing activity was measured by the test method shown below.

### [1. Test method]

### (1) Cells

As cells, B16 mouse melanoma cells (B16-F1) were used. Cells passaged 5-10 times, counting from the time of purchase, were used in the test. As medium, DMEM containing 5 or 10% FBS was used.

### (2) Melanin production suppression test

A quantity of 500 µL of DMEM medium containing 5% FBS was placed in a 24-well plate, and B16 melanoma cells were seeded 1.4 × 10⁴ cells/well (7.5 × 10⁴ cells/cm²), then cultured for 24 hours at 37°C under 5% CO₂. Thereafter, the medium was replaced with 1 mL of 0.5 mM theophylline-containing medium containing a test sample, and the cells were cultured for another three days.

As the test sample, a fermentation supernatant by each lactic acid bacterium or unfermented *Malva sylvestris* extract was added to make a blend ratio (volume ratio) of 0.5%, 1%, 2%, or 5% in the medium, taking the undiluted sample solution as 100%. As a control, RO water was added to the medium in the same blend ratios instead of the fermentation supernatant by each lactic acid bacterium or unfermented *Malva sylvestris* extract. As a positive control, 1% of an aqueous solution of arbutin, known as a whitening ingredient for quasi-drugs, was added to the medium (final concentration 0.15-2.44 mM).

### (3) Intracellular melanin production level

After culture was completed, the cells were washed twice with PBS(-) and fixed with 99.5% ethanol. After removing the ethanol by air drying, 1N NaOH was added to each well and heated for 30 minutes at 80°C. A cell lysate dissolving the cells and melanin was obtained. After allowing to cool, the entire amount of cell lysate was transferred to a 96-well plate, and the 405 nm absorbance was measured. The intracellular melanin production level per culture well was determined from a calibration curve that used synthetic melanin, and the intracellular melanin production level relative to the control was determined by the following formula. Intracellular melanin production level (%) = [intracellular melanin production level of test sample/intracellular melanin production level of control]× 100

### (4) Intracellular protein level

The cell lysate was diluted ten-fold by ultrapure water (milli-Q water) and assayed by the BCA method (Pierce BCA Protein assay kit) using BSA as the reference protein, and the intracellular protein level relative to the control was determined using the following formula.[Numerical formula 2] Intracellular protein level (%) = [intracellular protein level of test sample/intracellular protein level of control]× 100

### (5) IC50 value

The 50% inhibitory concentration (IC50 value) for each of the intracellular melanin production level and the intracellular protein level was determined based on 2-5 tests of fermentation supernatants by each lactic acid bacterium or unfermented *Malva sylvestris* extract and predetermined blend ratios. In calculation of the IC50 value, each value without test sample added was taken to be 100%, and a concentration making 50% or an estimated value thereof was used. Furthermore, the concentration of each fermentation supernatant and unfermented *Malva sylvestris* extract was expressed in terms of the evaporation residue concentration shown in Table 3.

### [2. Results]

The results are summarized in Table 4.

**[Table 4]**

| Sample No. | Type of sample | IC₅₀ µg/mL | |
|---|---|---|---|
| | | Intracellular melanin | Intracellular protein |
| 1 | Fermentation supernatant by lactic acid bacterium No. 1 | 531 | >1000* |
| 2 | Fermentation supernatant by lactic acid bacterium No. 2 | 528 | >1000* |
| 4 | Fermentation supernatant by lactic acid bacterium No. 4 | 685 | >1000* |
| 6 | Fermentation supernatant by lactic acid bacterium No. 6 | 650 | >1000* |
| 9 | Fermentation supernatant by lactic acid bacterium No. 9 | 1243 | >1000* |
| 10 | Fermentation supernatant by lactic acid bacterium No. 10 | 1032 | >1000* |
| 14 | *Malva sylvestris* extract | 1249 | >1000* |
| 15 | Arbutin (positive control) | 340 | 1200 |

| | | | |
|---|---|---|---|
| *: estimated value | | | |

As a result, while the positive control arbutin had an IC50 value for intracellular melanin production level of 340 µg/mL, the IC50 value of unfermented *Malva sylvestris* extract exceeded 1200 µg/mL, and the suppressive effect was poor.

Fermentation supernatants obtained using lactic acid bacterium No. 9 (*Leuconostoc pseudomesenteroides*) and lactic acid bacterium No. 10 *(Leuconostoc mesenteroides* subsp. *mesenteroides*), as with the unfermented *Malva sylvestris* extract, had a poor melanin production-suppressing effect.

Meanwhile, the IC50 value for intracellular melanin production level in fermentation supernatants obtained using lactic acid bacterium No. 1 (*Lactobacillus plantarum* subsp. *plantarum*), lactic acid bacterium No. 2 *(Lactobacillus pentosus),* lactic acid bacterium No. 4 (*Lactobacillus mali),* and lactic acid bacterium No. 6 (*Lactobacillus fabifermentans*) were remarkably lower than unfermented and tended to approach the value of the positive control arbutin. On the other hand, no major differences were seen from unfermented in the IC50 values of intracellular protein level in these fermentation supernatants.

It is clear from the above that the melanin production-suppressing activity is increased in fermentation products of *Malva sylvestris* extract obtained using the above lactic acid bacteria without causing a decrease in cell count in comparison to the unfermented extract.

### [Test Example 3]

Lactic acid bacteria Nos. 3, 6, 7, 8, 11, and 12 were selected among the 12 types of lactic acid bacteria (Nos. 1-12) that exhibited a viable cell count of 1 × 10⁶ CFU/mL or more after culture in Test Example 1. Fermentation supernatants were prepared in the same way as in Test Example 2 (the fermentation supernatant used in Test Example 2 was used for No. 6), and the tyrosinase-inhibiting activity was studied. Table 5 shows the results of pH measurement of the fermentation supernatants used or unfermented *Malva sylvestris* extract and the evaporation residue concentration (mg/mL) when measured by allowing to cool in a desiccator after heating for three hours at 105°C.

**[Table 5]**

| Sample No. | Type of sample | pH | Evaporation residue concentration* (mg/mL) |
|---|---|---|---|
| 3 | Fermentation supernatant by lactic acid bacterium No. 3 | 3. 9 | 21.4 |
| 6 | Fermentation supernatant by lactic acid bacterium No. 6 | 3. 9 | 21.2 |
| 7 | Fermentation supernatant by lactic acid bacterium No. 7 | 3. 9 | 21.1 |
| 8 | Fermentation supernatant by lactic acid bacterium No. 8 | 4.5 | 21.0 |
| 11 | Fermentation supernatant by lactic acid bacterium No. 11 | 4.0 | 20.0 |
| 12 | Fermentation supernatant by lactic acid bacterium No. 12 | 3. 9 | 19.8 |
| 14 | *Malva sylvestris* extract (unfermented) | 5.1 | 21.8 |

| | | | |
|---|---|---|---|
| Evaporation residue concentration*: allowed to cool in desiccator after heating for three hours at 105°C | | | |

The tyrosinase-inhibiting activity was measured by a partial modification of the method of Matsuda et al. (Matsuda H et al.: Studies of cuticle drugs from natural sources. III. Inhibitory effect of Myrica rubra on melanin biosynthesis, Biol. Pharm. Bull., 18(8), 1148-1150 (1995)). Specifically, the fermentation supernatant obtained using each lactic acid bacterium or unfermented *Malva sylvestris* extract was aliquoted in 50 µL portions (undiluted sample solution) into each well of 96-well microplates. After adding 50 µL of 300 mM phosphate buffer (pH 6.8) to each well and mixing, the plates were preincubated for ten minutes at room temperature. Next, 25 µL of 270 U/mL tyrosinase solution (tyrosinase: mushroom derived, Sigma-Aldrich) and 25 µL of 0.06 w/v% 3,4-dihydroxyL-phenylalanine (L-DOPA, FUJIFILM Wako Pure Chemical Corporation) were added to each well and mixed. After incubating for five minutes at room temperature, the 475 nm absorbance, which is the maximum absorption wavelength of dopachrome (intermediate of melanin biosynthesis), was measured. The final volume of the reaction system was 150 µL, of which the final blend ratio of fermentation supernatant (undiluted sample solution) was 50 µL/150 µL.

The tyrosinase inhibition rate was calculated using the following formula from the measured absorbance. In the formula, "control" represents a reaction system with RO water added instead of sample and "blank" represents a reaction system with 50 mM potassium phosphate buffer added instead of tyrosinase.[Numerical formula 3] Tyrosinase inhibition rate (%) ={1-[(S475 nm - SB475 nm) / (C475 nm - CB475 nm)]} × 100
S_{475 nm}: sample
SB_{475 nm}: sample blank
C_{475 nm}: control
CB_{475 nm}: control blank

The tyrosinase reaction test was carried out three times on fermentation supernatants obtained using each lactic acid bacterium or unfermented *Malva sylvestris* extract, and the mean and standard deviation were determined. The results are shown in Table 6.

**[Table 6]**

| Sample No. | Type of sample | Tyrosinase inhibition rate | |
|---|---|---|---|
| | | Mean* | ±SD* |
| 3 | Fermentation supernatant by lactic acid bacterium No. 3 | 6% | 1% |
| 6 | Fermentation supernatant by lactic acid bacterium No. 6 | 8% | 3% |
| 7 | Fermentation supernatant by lactic acid bacterium No. 7 | 4% | 4% |
| 8 | Fermentation supernatant by lactic acid bacterium No. 8 | 24% | 1% |
| 11 | Fermentation supernatant by lactic acid bacterium No. 11 | 21% | 1% |
| 12 | Fermentation supernatant by lactic acid bacterium No. 12 | 17% | 2% |
| 14 | *Malva sylvestris* extract (unfermented) | 2% | 8% |

| | | | |
|---|---|---|---|
| Mean*±SD*: n = 3 | | | |

In the results, while the tyrosinase inhibition rate of the unfermented *Malva sylvestris* extract was 2% (SD: ±8%), among fermentation products by each lactic acid bacterium the inhibition rate was 6% (SD: ±1%) with lactic acid bacterium No. 3 *(Lactobacillus zeae),* 8% (SD: ±3%) with lactic acid bacterium No. 6 (*Lactobacillus fabifermentans*), 4% (SD: ±4%) with No. 7 (*Lactobacillus hordei*), 24% (SD: ±1%) with lactic acid bacterium No. 8 *(Lactococcus lactis* subsp. *lactis), 21%* (SD: ±1%) with lactic acid bacterium No. 11 (*Pediococcus acidilactici*), and 17% (SD: ±2%) with lactic acid bacterium No. 12 (*Pediococcus pentosaceus*).

From the above it is clear that the tyrosinase-inhibiting activity is increased in fermentation products of *Malva sylvestris* extract obtained using the above lactic acid bacteria in comparison to the unfermented extract.

### [Test Example 4]

An investigation was made into whether the functionalities of other genus of Malvaceae plants are enhanced by lactic acid bacterial treatment, as with *Malva sylvestris.*

### [4-1. Plants]

In addition to the dried flowers of *Malva sylvestris* used in Test Examples 1-3, dried calices and bracts of hibiscus and dried flowers of black mallow were used in the test. Table 7 shows the genus name, scientific name, Japanese name, and English common name of the Malvaceae used.

**[Table 7]**

| Malvaceae plant | Part | Genus name | Scientific name | Japanese name | English common name |
|---|---|---|---|---|---|
| *Malva sylvestris* | Flowers | Malva | *Malva sylvestris* | Ueubeniaoi | Blue mallow/ common mallow |
| Hibiscus | Calices and bracts | Hibiscus | *Hibiscus sabdariffa* | Roozerusou | Hibiscus |
| Black mallow | Flowers | Alcea | *Alcea rosea* | Tachiaoi | Hollyhock |

### [4-2. Preparation of plant extract]

A plant extract for preculture was prepared as follows. Water (reverse osmosis-treated water, referred to hereinafter as "RO water") was added to achieve a plant powder:water ratio of 1:20 (mass). Glucose was added to yield a final concentration of 0.2 w/v% and yeast extract (Difco) was added to yield a final concentration of 0.1 w/v%. A suspension was prepared via thorough stirring. The suspension was aliquoted in 3 mL portions into test tubes which were then covered with an aluminum cap and autoclaved for 15 minutes at 121°C to obtain a hot-water extract.

A plant extract for main culture was prepared as follows. 12 mL of RO water was added so that the plant powder:water ratio was 1:20 (mass) in the case of *Malva sylvestris* and hibiscus, or so that the plant powder:water ratio was 1:50 (mass) in the case of black mallow. A suspension was prepared via thorough stirring. The suspension was placed in test tubes which were sealed with a silicone plug and autoclaved for 100 minutes at 98°C to obtain a hot-water extract.

### [4-3. Lactic acid bacteria]

Lactic acid bacterium No. 1 (*Lactobacillus plantarum* subsp. *plantarum*) was used.

### [4-4. Preculture and main culture]

Preculture and main culture obtained using lactic acid bacterium No. 1 were carried out in the same way as in Test Example 1 except that the hot-water extracts prepared above were used as the culture starting material.

### [4-5. Confirmation of viable cell count after main culture and pH and evaporation residue concentration of fermentation supernatant]

The viable cell count after the main culture was confirmed in the same way as in Test Example 1. Also, a fermentation supernatant was prepared in the same way as in Test Example 2 from the culture after the main culture had been completed, and the pH and evaporation residue concentration thereof were confirmed.

Table 8 summarizes these results.

**[Table 8]**

| Malvaceae plant | Fermentation | Viable cell count (CFU (×10⁶/mL) | pH | Evaporation residue concentration* (mg/mL) |
|---|---|---|---|---|
| *Malva sylvestri*s | Unfermented | <10 | 4.9 | 21.3 |
| | Lactic acid bacterium No. 1 | 55 | 3.9 | 19.6 |
| Hibiscus | Unfermented | <10 | 5.4 | 35.6 |
| | Lactic acid bacterium No. 1 | 119 | 4.5 | 35.2 |
| Black mallow | Unfermented | <10 | 5.1 | 10.1 |
| | Lactic acid bacterium No. 1 | 156 | 3.5 | 7.5 |

| | | | | |
|---|---|---|---|---|
| Evaporation residue concentration*: allowed to cool in desiccator after heating for three hours at 105°C | | | | |

### [4-6. Melanin production-suppressing activity]

The melanin production-suppressing activity of the obtained fermentation supernatants or unfermented plant extract was measured using B16 melanoma cells in the same way as in Test Example 2. Taking the undiluted sample solution as 100%, samples were added to make blend ratios of 0.5%, 1%, 2%, and 5% (volume) in the medium. RO water was added to the medium in the same blend ratio as a control.

Table 9 summarizes the results on melanin production-suppressing activity for each Malvaceae plant extract, whether fermented by lactic acid bacteria or not, and at each blend ratio (volume ratio) of undiluted sample solution added to the medium. The data in the upper part of Table 9 (Table 9-1) are given in terms of relative values, taking the intracellular melanin level when the control (RO water) was added as 100%, and the data in the lower part of Table 9 (Table 9-2) are given in terms of absolute values, taking the intracellular melanin production level when unfermented plant extract was added in the same final concentration as 100%. In FIG. 1, the results of the lower part of Table 9 (Table 9-2) are graphed by expressing the final concentration of sample added to the medium in terms of the evaporation residue concentration and using the same on the x axis.

**[Table 9]**

| [Table 9-1] | | | | | | | | | |
|---|---|---|---|---|---|---|---|---|---|
| | (Intracellular melanin production level) | | | | | | | | |
| Malvaceae plant | Fermentation | Proportion added to medium (volume ratio) | | | | | | | |
| | | 0.5% | | 1.0% | | 2.0% | | 5% | |
| | | Mean* | ±SD* | Mean* | ±SD* | Mean* | ±SD* | Mean* | ±SD* |
| *Malva sylvestris* | Unfermented | 96% | 5% | 90% | 4% | 76% | 5% | 50% | 10% |
| | Lactic acid bacterium No. 1 | 88% | 10% | 71% | 5% | 56% | 3% | 31% | 11% |
| Hibiscus | Unfermented | 91% | 4% | 84% | 8% | 82% | 3% | 70% | 2% |
| | Lactic acid bacterium No. 1 | 85% | 7% | 80% | 8% | 75% | 4% | 69% | 2% |
| Black mallow | Unfermented | 100% | 7% | 98% | 7% | 93% | 7% | 76% | 2% |
| | Lactic acid bacterium No. 1 | 99% | 6% | 94% | 4% | 90% | 2% | 67% | 2% |

| | | | | | | | | | |
|---|---|---|---|---|---|---|---|---|---|
| Mean*±SD*: n = 3 | | | | | | | | | |

**[Table 9-2]**

| (Intracellular melanin production level-taking unfermented as 100%) | | | | | | | | | |
|---|---|---|---|---|---|---|---|---|---|
| Malvaceae plant | Fermentation | Proportion added to medium (volume ratio) | | | | | | | |
| | | 0.5% | | 1.0% | | 2.0% | | 5% | |
| | | Mean* | ±SD* | Mean* | ±SD* | Mean* | ±SD* | Mean* | ±SD* |
| *Malva sylvestris* | Unfermented | 100% | 0% | 100% | 0% | 100% | 0% | 100% | 0% |
| | Lactic acid bacterium No. 1 | 91% | 7% | 79% | 2% | 74% | 2% | 60% | 11% |
| Hibiscus | Unfermented | 100% | 0% | 100% | 0% | 100% | 0% | 100% | 0% |
| | Lactic acid bacterium No. 1 | 94% | 7% | 95% | 3% | 91% | 2% | 98% | 5% |
| Black mallow | Unfermented | 100% | 0% | 100% | 0% | 100% | 0% | 100% | 0% |
| | Lactic acid bacterium No. 1 | 99% | 4% | 96% | 4% | 97% | 10% | 88% | 3% |

| | | | | | | | | | |
|---|---|---|---|---|---|---|---|---|---|
| Mean*±SD*: n = 3 | | | | | | | | | |

In the results, as was also shown in Test Example 2, the fermentation supernatant of a *Malva sylvestris* extract treated by lactic acid bacteria had higher melanin production-suppressing activity than the unfermented extract. In contrast, such enhancement of functionality by lactic acid bacterial treatment was not seen in hibiscus or black mallow, which are other Malvaceae plants.

## Claims

1. A fermentation product having *Malva sylvestris* or a processed product thereof as a starting material, obtained using one or more microorganisms selected from the group consisting of microorganisms belonging to the genus Lactobacillus, microorganisms belonging to the genus Lactococcus, and microorganisms belonging to the genus Pediococcus.

2. The fermentation product according to Claim 1, wherein the microorganism is one or more selected from the group consisting of *Lactobacillus plantarum, Lactobacillus pentosus, Lactobacillus zeae, Lactobacillus mali, Lactobacillus fabifermentans, Lactobacillus hordei, Lactococcus lactis, Pediococcus acidilactici,* and *Pediococcus pentosaceus.*

3. A topical skin agent containing the fermentation product according to Claims 1 or 2.

4. The topical skin agent according to Claim 3, used to cause a whitening effect.

5. The topical skin agent according to Claims 3 or 4, wherein the fermentation product has a melanin production-suppressing action.

6. The topical skin agent according to any of Claims 3 to 5, wherein the fermentation product has a tyrosinase-inhibiting action.

## Patentansprüche

1. Fermentationsprodukt, das Malva sylvestris oder ein verarbeitetes Produkt daraus als Ausgangsmaterial aufweist, erhalten unter Verwendung eines oder mehrerer Mikroorganismen, die aus der aus Mikroorganismen, die zum Genus Lactobacillus gehören, Mikroorganismen, die zum Genus Lactococcus gehören, und Mikroorganismen, die zum Genus Pediococcus gehören, bestehenden Gruppe ausgewählt sind.

2. Fermentationsprodukt nach Anspruch 1, wobei der Mikroorganismus einer oder mehrere ist, die aus der aus Lactobacillus plantarum, Lactobacillus pentosus, Lactobacillus zeae, Lactobacillus mali, Lactobacillus fabifermentans, Lactobacillus hordei, Lactococcus lactis, Pediococcus acidilactici und Pediococcus pentosaceus bestehenden Gruppe ausgewählt sind.

3. Topisches Hautmittel, das ein Fermentationsprodukt nach Anspruch 1 oder 2 enthält.

4. Topisches Hautmittel nach Anspruch 3, das für einen Aufhellungseffekt verwendet wird.

5. Topisches Hautmittel nach Anspruch 3 oder 4, wobei das Fermentationsprodukt eine Melaninproduktion-unterdrückende Wirkung aufweist.

6. Topisches Hautmittel nach einem der Ansprüche 3 bis 5, wobei das Fermentationsprodukt eine Tyrosinase-inhibierende Wirkung aufweist.

## Revendications

1. Produit de fermentation ayant de la *Malva sylvestris* ou un produit traité de celle-ci comme matériau de départ, obtenu en utilisant un ou plusieurs micro-organismes choisis dans le groupe constitué de micro-organismes appartenant au genre Lactobacillus, de micro-organismes appartenant au genre Lactococcus, et de micro-organismes appartenant au genre Pediococcus.

2. Produit de fermentation selon la revendication 1, dans lequel le micro-organisme est un ou plusieurs choisis dans le groupe constitué de *Lactobacillus plantarum, Lactobacillus pentosus, Lactobacillus zeae, Lactobacillus mali, Lactobacillus fabifermentans, Lactobacillus hordei, Lactococcus lactis, Pediococcus acidilactici,* et *Pediococcus pentosaceus.*

3. Agent topique pour la peau contenant le produit de fermentation selon les revendications 1 ou 2.

4. Agent topique pour la peau selon la revendication 3, utilisé pour provoquer un effet de blanchiment.

5. Agent topique pour la peau selon la revendication 3 ou 4, dans lequel le produit de fermentation présente une action de suppression de la production de mélanine.

6. Agent topique pour la peau selon l'une quelconque des revendications 3 à 5, dans lequel le produit de fermentation présente une action d'inhibition de tyrosinase.
